# EUROPÄISCHE PATENTSCHRIFT

(11) **EP 0 414 274 B1**
(45) Veröffentlichungstag und Bekanntmachung des Hinweises auf die Patenterteilung: **23.06.1993**
(21) Anmeldenummer: 90116292.5
(22) Anmeldetag: 24.08.1990
(51) Int. Cl.: C07C 229/28, C07C 227/22, C07C 227/18

(54) **Verfahren zur Herstellung von 1-Aminomethyl-1-cyclohexanessigsäure**
Process for the preparation of 1 aminomethyl-1.cyclohexanacetic acid
Procédé de préparation de l'acide 1-aminomethyl-1.cyclohexaneacetique

(30) Priorität: 25.08.1989 DE 3928182
(43) Veröffentlichungstag der Anmeldung: 27.02.1991
(73) Patentinhaber: GÖDECKE AKTIENGESELLSCHAFT, D-10587 Berlin (DE)
(72) Erfinder: Geibel, Wolfram, Dr., D-6418 Hünfeld (DE); Hartenstein, Johannes, Dr., D-7801 Stegen-Wittental (DE); Herrmann, Wolfgang, Dr., D-7802 Merzhausen (DE); Witzke, Joachim, Dr., D-7835 Nimburg (DE)

(56) Entgegenhaltungen:
- DE-A- 2 460 891
- DE-A- 2 626 467
- GB-A- 825 562
- US-A- 2 812 334
- US-A- 3 624 127
- JOURNAL OF THE AMERICAN CHEMICAL SOCIETY. vol. 83, no. 7, 19 April 1961, GASTON, PA US Seiten 1733 - 1738; W.S. WADSWORTH ET AL.: "THE UTILITY OF PHOSPHONATE CARBANIONS IN OLEFIN SYNTHESIS"
- ORGANIC PREPARATIONS AND PROCEDURES INTERNATIONAL vol. 19, no. 6, 1987, NEWTON, MASS. Seiten 471 - 475; RICHARD A. BUNCE ET AL.: "MICHAEL REACTION OF NITROMETHANE WITH BETA,BETA-DISUBSTITUTED ACRYLATE ESTERS."

## Beschreibung

Gabapentin (1-Aminomethyl-1-cyclohexanessigsäure) ist ein in den DE-OS 24 60 891 und 25 43 821 beschriebener Arzneimittelwirkstoff zur Therapie bestimmter zerebraler Erkrankungen wie beispielsweise Epilepsie und Schwindelanfälle.

Im genannten Stand der Technik werden zur Herstellung von Gabapentin und verwandten Verbindungen verschiedene Verfahren zu deren Herstellung aufgeführt.

Beispielsweise kann Gabapentin hergestellt werden, indem 1,1-Cyclohexandiessigsäure über ein reaktives Säurederivat in das Azid überführt und dieses anschließend durch thermische Zersetzung dem Curtiusschen Abbau unterworfen wird.

Das Verfahren ist für eine technische Anwendung aus Gründen der Sicherheit weniger geeignet, da das gebildete Azid beim Erhitzen oder beim Bearbeiten (Berührung mit Säure) leicht explodieren kann.

Nach den bekannten Verfahren wird beispielsweise auch durch Lossen-Abbau der entsprechenden Hydroxamsäure Gabapentin erhalten.

Das bisher zur Herstellung von Gabapentin über den Lossen-Abbau bekannte Verfahren läuft in 8 Reaktionsstufen ab:

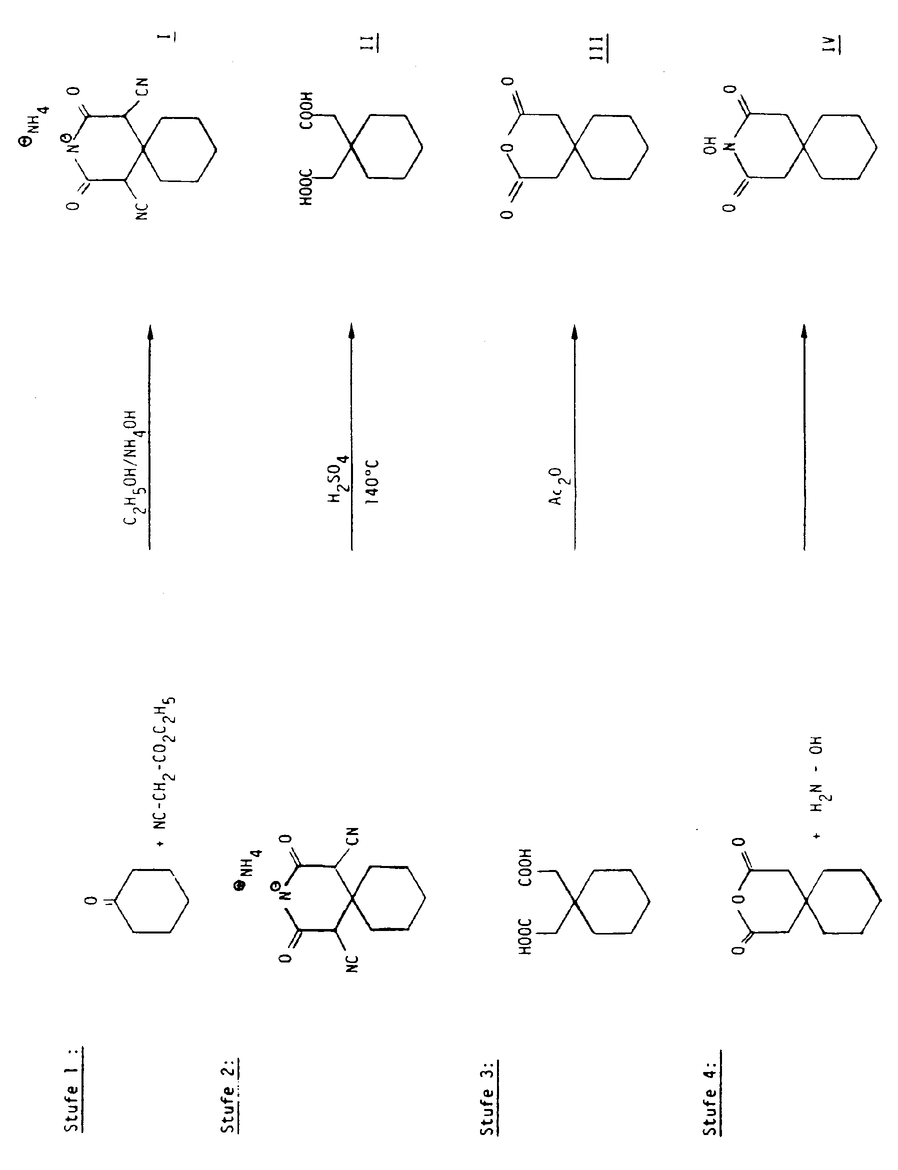

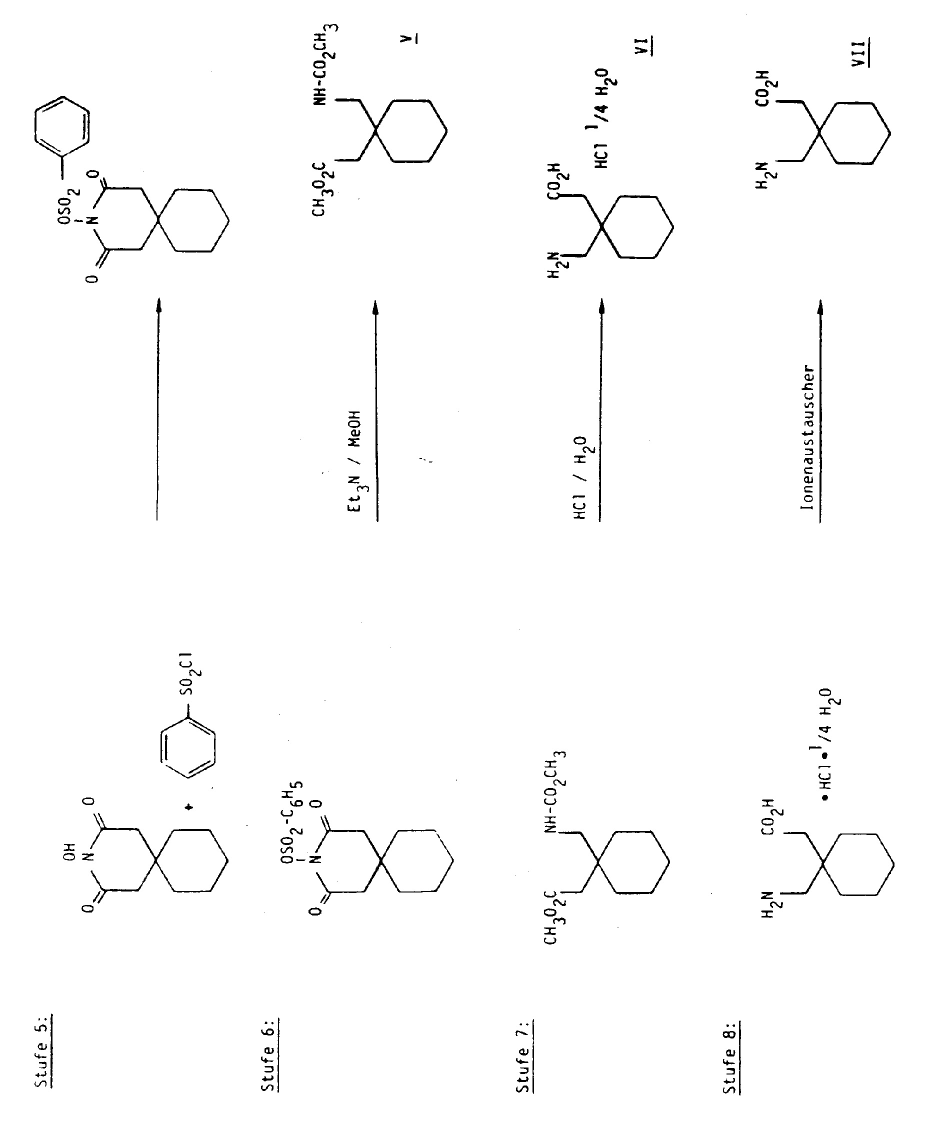

Das Verfahren nach dem Stand der Technik besitzt einige gravierende Nachteile, auf die kurz eingegangen werden soll:
- Die Gesamtausbeute über alle Stufen beträgt nur 30%.
- Wegen der vielen Reaktionsstufen bedarf das Verfahren eines relativ großen Zeit- und Arbeitsaufwandes, daraus resultiert ein entsprechend hoher Herstellungspreis.
- Im Reaktionsschritt 1 wird mit großen Mengen gasförmigen Ammoniaks bei niedrigen Temperaturen gearbeitet.
- Im Reaktionsschritt 2 wird als Lösungsmittel 80%ige Schwefelsäure bei 160°C verwendet. Die Vernichtung dieser und weiterer im Herstellungsprozeß eingesetzter Säuren führt zu einer hohen Abwasserbelastung.
- Das Verfahren erfordert teilweise teure Hilfsreagenzien, welche nicht ins Produkt eingebaut werden und somit wesentlich zu den hohen Herstellungskosten beitragen.

Es stellte sich also die Aufgabe, für die Herstellung von Gabapentin ein neues Herstellungsverfahren zu entwickeln, welches folgende Bedingungen erfüllen sollte:
1. Das Verfahren sollte weniger Reaktionsstufen umfassen und mit geringerem Zeit- und Arbeitsaufwand durchführbar sein.
2. Die Gesamtausbeute sollte deutlich über der des bekannten Verfahrens liegen.
3. Die Herstellungskosten sollten nach Möglichkeit die des bekannten Verfahrens unterschreiten.
4. Das Verfahren sollte im technischen Maßstab durchführbar sein.
5. Das Verfahren sollte eine minimale Gefährdung von Personen und eine verringerte Belastung der Umwelt gewährleisten.

Gegenstand der vorliegenden Erfindungsmeldung ist ein neues, optimiertes Verfahren zur Herstellung von Gabapentin nach dem Prinzip der Michael-Addition, welches die obigen Anforderungen erfüllt. Das neue Verfahren ist durch folgende Reaktionsschritte gekennzeichnet:
a.) Umsetzung von Cyclohexanon mit einer Phosphonsäureester-Verbindung zu Cyclohexylidenessigsäureester
b.) Umsetzung des Cyclohexylidenessigsäureesters mit Nitromethan zu 1-Nitromethyl-cyclohexanessigsäureester
c.) Reduktion des 1-Nitromethyl-cyclohexanessigsäureesters zu 1-Aminomethyl-cyclohexanessigsäureester und 2-Aza-Spiro [4,5] decan-3-on
d.) Umwandlung der im Schritt c.) entstandenen Produkte in 1-Aminomethyl-1-cyclohexanessigsäure-Salze mittels verdünnter Säuren.
e.) Freisetzung der 1-Aminomethyl-1-cyclohexanessigsäure aus dem Salz mittels Ionenaustauschern,
   wobei in Schritt a.) zunächst das Cyclohexanon bei Raumtemperatur mit KOH umgesetzt wird, bevor der Phosphonsäureester zugegeben wird,
   in Schritt b.) DMSO als Lösungsmittel und ein Alkalimetallcarbonat als Katalysator verwendet wird
   in Schritt c.) bei erhöhter Temperatur, nämlich oberhalb 100°C gearbeitet wird.

Als Cyclohexylidenessigsäureester kommen 1-6 C Alkylester, bevorzugt der Ethylester, in Frage.

Das erfindungsgemäße Verfahren ermöglicht eine einfache, schnelle und kostengünstige Herstellung von Gabapentin. Das Herstellungsverfahren besteht nur mehr aus 5 Reaktionsstufen und ergibt eine Gesamtausbeute von ca. 50% gegenüber 30% beim bekannten Verfahren. Durch Rückführung nicht umgesetzten Ausgangsmaterials im Reaktionsschritt 4 in den Reaktionsprozeß ist die Ausbeute nochmals um bis zu 15% zu steigern.

Auch bezüglich der technischen Durchführbarkeit ist das erfindungsgemäße Verfahren durch kurze und einfache Herstellungsprozesse dem bekannten Verfahren überlegen. Alle Reaktionen sind nach maximal 4 Stunden abgeschlossen. Die Umsetzung der Stufe 1 erfolgt bei Raumtemperatur. Alle Reaktionsprodukte werden aus der Reaktionsmischung extrahiert oder durch Entfernen des Lösungsmittels direkt erhalten. Die isolierte Stufe 4 ist nach HPLC rein und die Reinheit der übrigen Stufen ist größer als 95%, so daß keinerlei Reinigungsoperationen notwendig sind.

Das neue optimierte Verfahren nach der Erfindung ist nachstehend formelmäßig beschrieben.

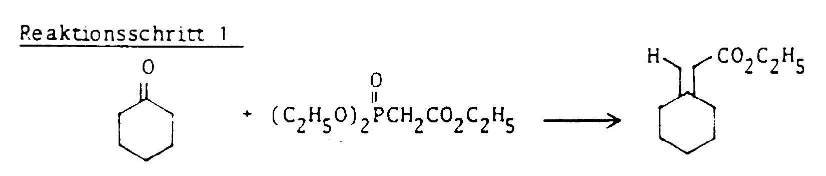

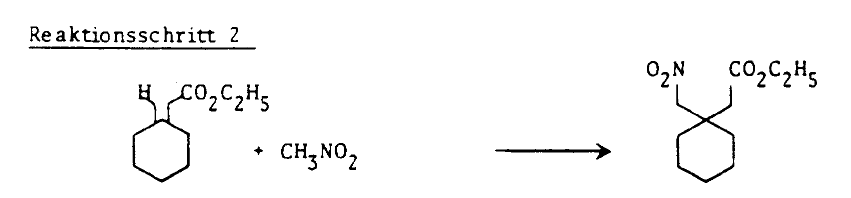

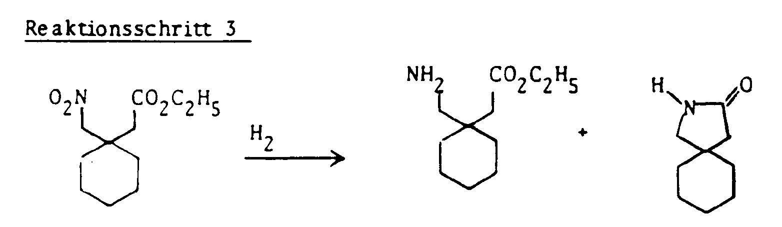

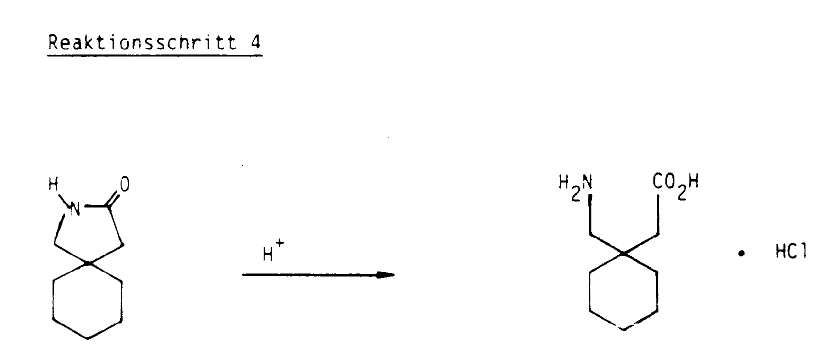

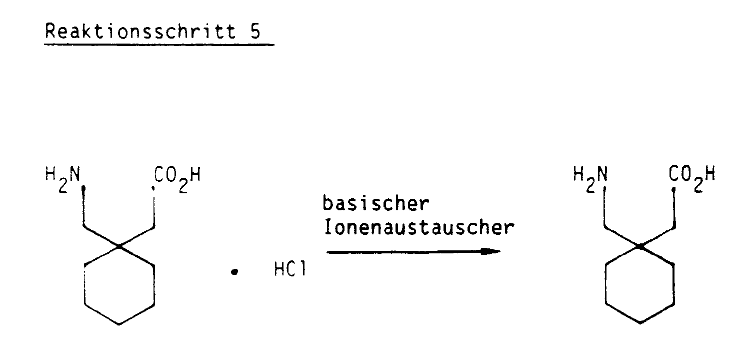

Im Reaktionsschritt 1 des erfindungsgemäßen Verfahrens wird Cyclohexanon zu Cyclohexylidenessigsäureester umgesetzt. Üblicherweise wird dazu zuerst die phosphororganische Verbindung mit einer Base wie z.B. Natriumhydrld und anschließend mit Cyclohexanon umgesetzt.
An Stelle von Natriumhydrid kann NaOH oder auch KOH verwendet und sicherer gehandhabt werden, jedoch treten hierbei Ausbeuteverluste und Nebenprodukte (Esterspaltung und Zersetzung) auf.
Überraschenderweise wurde gefunden, daß durch einen geänderten Reaktionsablauf auch unter Verwendung von KOH die Produktausbeute und Reinheit stark gesteigert und die Bildung von Nebenprodukten vermieden werden kann. Dadurch ist es möglich geworden, auf aufwendigere Reinigungsoperationen (Vakuum-Destillation) zu verzichten. Bei diesem geänderten Reaktionsablauf wird zuerst Cyclohexanon bei Raumtemperatur mit KOH umgesetzt, bevor Phosphonsäureester zugegeben wird. Nach kurzer Nachreaktion bei Raumtemperatur wird die Reaktion durch Zugabe von Wasser beendet und das Produkt mit Petroläther aus der wässrigen Lösung extrahiert. Nach Entfernen des Lösungsmittels fällt das Produkt mit über 90% Ausbeute und einer Reinheit von 98% an.

Im Reaktionsschritt 2 des erfindungsgemäßen Verfahrens findet die Michael-Addition von Nitromethan an Stufe 1 unter Basenkatalyse statt.

Die Addition von Nitromethan an β,β-disubstituierte Acrylsäureester wie Stufe 1 (Cyclohexylidenessigsäureester) ist wegen der ungenügenden Aktivierung durch eine Estergruppe und der sterischen Hinderung der Substituenten erschwert. Es ist Stand der Technik, bei wenig aktivierten Reaktionspartnern Nitromethan in großem Überschuß mit oder ohne Lösungsmittel meist unter Rückfluß einzusetzen.

J. Org. Chem. 50, 2806 (1985) beschreibt Michael-Additionen an Acrylsäureester ohne Nitromethanüberschuß. Jedoch ist dazu vorab die stöchiometrische Bildung des Salzes aus Nitromethan mit Basen wie KOH oder K-tert. Butylat erforderlich. Außerdem ist bei den beschriebenen β,β-disubstituierten Verbindungen die Doppelbindung durch eine weitere elektronenziehende Gruppe aktiviert.

Die Michael-Addition erfordert die Gegenwart basischer Katalysatoren wie beispielsweise KOH, verschiedene Amine, Alkoholate, Metallfluoride usw. Auch Tetraalkylammoniumfluoride sind sehr effektive Katalysatoren für die Michael-Addition. Unter den genannten Bedingungen, d. h. mit einem großen Überschuß an Nitromethan ist die beschriebene Reaktion mit Stufe 1 im Labormaßstab durchführbar, wobei Tetraäthylammoniumfluorid oder KOH die Katalysatoren der Wahl sind. Für das erfindungsgemäße Verfahren waren diese Bedingungen jedoch nicht anwendbar, da Nitromethan bekannterweise mit starken Basen explosionsfähige Verbindungen bildet oder dazu befähigt wird, mit Hilfe von Initialzündern zu explodieren.
Nitromethan existiert in einem tautomeren Gleichgewicht mit der entsprechenden Nitronsäure. Nitronsäure bildet mit KOH ein extrem instabiles, hoch schlagempfindliches Salz. Amine wiederum ergeben eine detonationsempfindliche Mischung. Allgemein sind alkalische Lösungen von Nitromethan bekanntlich nicht stabil, wenn sie erhitzt oder länger aufbewahrt werden.

Ein weiterer Grund für die Nichtanwendbarkeit obiger Bedingungen auf das erfindungsgemäße Verfahren besteht einmal in der durch starke Basen bewirkten Spaltung der Stufe 1 zur Carbonsäure und zum anderen in der Tatsache, daß in diesem speziellen Fall starke Basen (KOH) und Tetraalkylammoniumfluoride die Verlagerung der Doppelbindung in den Ring katalysieren. Die Bildung der Verbindung mit in den Ring verlagerter Doppelbindung ist fast quantitativ. Sie ist thermodynamisch gegenüber der Stufe 1 mit Konjugation von Doppelbindung und Esterfunktion bevorzugt. Bei Abwesenheit von Nitromethan setzt sich die Stufe 1 unter Basenkatalyse in weniger als 15 min zum stabileren Nebenprodukt 1-Cyclohexen-essigsäureester um. In Gegenwart von äquimolaren Mengen Nitromethan ist die Nebenproduktbildung gegenüber der Bildung von Stufe 2 immer noch bevorzugt und man erhält nach dem Aufarbeiten nur ca 10 % Stufe 2 und ca 90 % 1-Cyclohexen-essigsäureester.

Wegen des Gefahrenpotentials von Nitromethan einerseits und den unerwünschten Nebenreaktionen andererseits mußte versucht werden, a) die Verwendung starker Basen zu vermeiden, b) den Anteil Nitromethan möglichst auf den zur Reaktion notwendigen Anteil, d.h. auf ein Äquivalent, zu reduzieren.
Nach vorhandenen Erfahrungen war nicht zu erwarten, daß die Reaktion in guten Ausbeuten ablaufen würde, wenn der Nitromethananteil auf das erforderliche Mindestmaß reduziert wird, schwache Basen als Katalysator verwendet werden und ein zusätzliches Lösungsmittel eingesetzt wird.
Es wurde überraschenderweise gefunden, daß im Reaktionsschritt 2 trotz der eingeschränkten Bedingungen Ausbeuten von 90% und Reinheiten von ≧ 90% erzielbar sind und keine Nebenprodukte gebildet werden, wenn DMSO als Lösungsmittel verwendet wird in Verbindung mit katalytischen Mengen (10-50 Mol%) K₂CO₃ oder Na₂CO₃ als basischen Katalysator. Im einzelnen wird so vorgegangen, daß Nitromethan in äquimolaren Mengen (mit 10-50% Überschuß) zusammen mit Stufe 1 langsam, kontinuierlich zum Reaktionsgemisch aus Lösungsmittel und Katalysator bei 95-100°C zugegeben wird. Ebenso kann aber auch Nitromethan allein kontinuierlich zum Reaktionsgemisch aus Lösungsmittel, Katalysator und Stufe bei 95-100°C zugegeben werden. Jedoch muß hierbei mit der Bildung von Nebenprodukten gerechnet werden. In jedem Fall wird durch die kontinuierliche Nitromethanzugabe sichergestellt, daß Nitromethan immer abreagiert und sich zu keiner Zeit in größeren Mengen anreichern kann, woraus eine Gefahrenpotential resultieren könnte.

Der dritte Reaktionsschritt des erfindungsgemäßen Verfahrens beschreibt die katalytische Reduktion der Nitrogruppe zur Aminogruppe. Dazu wird das Produkt des Reaktionsschrittes 2 mit Wasserstoff an Edelmetallkatalysatoren wie Palladium auf Aktivkohle umgesetzt. Als Lösungsmittel kann Essigsäure, Äthanol oder auch Äthanol mit wässriger Salzsäure verwendet werden. Aus dieser Reaktion resultieren normalerweise 2 Produkte, das cyclisierte Produkt (Lactam) und 1-Aminomethyl-1-cyclohexanessigsäureäthylester. Wir fanden, daß bei ausreichend hoher Temperatur, bevorzugt bei 100 - 125°C, ausschließlich "Lactam" in mehr als 90% Ausbeute erhalten wird. Nach Entfernung des Katalysators und des Lösungsmittels wird das Lactam ohne weitere Reinigung im 4. Reaktionsschritt des erfindungsgemäßen Verfahrens mit wässriger Salzsäure in der Siedehitze zu Gabapentin-Hydrochlorid hydrolysiert. In Abhängigkeit von der Säurekonzentration wird eine Umwandlung in bis zu 80 % Gabapentin·HCl erzielt. Der nicht umgesetzte Anteil Lactam wird durch Extraktion zurückgewonnen und erneut der Hydrolyse unterworfen. Aus der verbleibenden wässrigen Lösung wird Gabapentin·HCl in 60-70 % Ausbeute mit 99 % Reinheit erhalten.

Die erzielte Reinheit macht eine weitere Reinigung überflüssig, so daß im Reaktionsschritt 5 des erfindungsgemäßen Verfahrens aus dem Hydrochlorid direkt die freie Aminosäure Gabapentin gewonnen werden kann. Dazu wird eine wässrige Lösung (20 %) von Gabapentin·HCl über eine mit schwach basischem Anionenaustauscher gefüllte Säule gegeben. Aus der resultierenden wässrigen Lösung wird durch schonendes Eindampfen im Vakuum und anschließende Kristallisation aus Methanol Gabapentin mit hoher Reinheit in 80-90 % Ausbeute erhalten.

In einem besonders bevorzugten Reaktionsschritt 1 wird Cyclohexanon und der Phosphonsäureester vorgelegt und in diese homogene Phase unter Rühren KOH eingetragen. In einer schnellen, exothermen Reaktion, die durch Kühlung und Dosiergeschwindigkeit der KOH auf 30-40°C gehalten wird, ist nach Ende der KOH-Zugabe die Umsetzung praktisch vollständig.

Außerdem hat sich herausgestellt, daß die katalytische Hydrierung in Ethanol als Lösungsmittel schon bei Temperaturen oberhalb 60°C schnell und praktisch vollständig zum Lactam führt.

### Beispiel 1

### Herstellung von Cyclohexylidenessigsäureäthylester (Stufe 1)

70,2 g (1,10 Mol) Kaliumhydroxid-Pulver (Gehalt 88%) werden in 168 ml Tetrahydrofuran suspendiert und unter Kühlung bei 20-25°C tropfenweise mit 98,2 g (1,0 Mol) Cyclohexanon versetzt. Man läßt 15 Minuten nachrühren und tropft anschließend bei Raumtemperatur 246,6 g (1,10 Mol) Phosphonoessigsäuretriäthylester während 15 - 30 Minuten zu und rührt eine Stunde bei Raumtemperatur nach.
Anschließend wird das Reaktionsgemisch mit Wasser versetzt und die organische Phase abgetrennt. Die wässrige Phase wird nochmals mit Methylenchlorid ausgeschüttelt. Die vereinigten organischen Phasen werden mit Wasser gewaschen, über Natriumsulfat getrocknet und dann im Vakuum das Lösungsmittel abdestilliert.
Man erhält als Öl 158,7 g Cyclohexylidenessigsäureäthylester (94,3% der Theorie)
Gehalt nach HPLC und GC 98-99%

### Beispiel 2

### Herstellung von 1-Nitromethyl-cyclohexanessigsäureäthylester (Stufe 2)

Zu einem Gemisch aus 64,72 g (0,469 Mol) Kaliumcarbonat in 469,5 ml Dimethylsulfoxid suspendiert, wird während 1-2 Stunden bei 95°C eine Lösung aus 158 g (0,939 Mol) Cyclohexylidenessigsäureäthylester und 85,94 g (1,408 Mol) Nitromethan zudosiert. Nach beendeter Zugabe wird bei 95°C 2-3 Stunden nachgerührt. Unter Eiswasserkühlung wird anschließend die Reaktionslösung mit ca. 150 ml konz. Salzsäure angesäuert und mit 1.5 l Wasser verdünnt. Die entstehenden Phasen werden getrennt und die wässrige Phase wird mehrmals mit Petroläther ausgeschüttelt. Die vereinigten organischen Phasen werden mit Wasser neutral gewaschen und über Natriumsulfat getrocknet. Das Lösungsmittel wird anschließend in Vakuum abdestilliert.
Man erhält als Öl 190,3 g 1-Nitromethyl-cyclohexanessigsäureäthylester (88,4% der Theorie).
Zusammensetzung nach HPLC: 90% Stufe 2 und 6% Cyclohexylidenessigsäureäthylester (Stufe 1)

### Beispiel 3

16,8 g (0,1 Mol) Cyclohexylidenessigsäureäthylester und 13,8 g (0,1 Mol) Kaliumcarbonat werden in 50 ml DMSO auf 95-100°C erwärmt und über 1 Stunde mit einer Lösung von 9,2 g (0,15 Mol) Nitromethan in 10 ml DMSO versetzt. Nach 1 Stunde Nachreaktion wird analog Beispiel 2 aufgearbeitet. Man erhält 19,26 g Produkt, enthaltend 61,4% 1-Nitromethyl-cyclohexanessigsäureäthylester, 27,5% Ausgangsverbindung und 7,8% Nebenprodukt (1-Cyclohexen-essigsäureäthylester).

### Beispiel 4

16,8 g (0,1 Mol) Cyclohexylidenessigsäureäthylester, 9,2 g (0,15 Mol) Nitromethan und 0,64 g (0,01 Mol) KOH (Gehalt 88%) in 40 ml DMSO werden 2 Stunden bei 100°C gerührt. Man arbeitet analog Beispiel 2 auf und erhält 20,5 g 1-Nitromethyl-cyclohexanessigsäureäthylester (89,4% der Theorie). Der Gehalt nach GC beträgt 82,8%, daneben sind noch 10,2% Ausgangsverbindung und 4,1% Nebenprodukt (siehe Beispiel 3) enthalten.

### Beispiel 5

16,8 g (0,1 Mol) Cyclohexylidenessigsäureäthylester und 0,64 g (0,01 Mol) KOH (Gehalt 88%) in 40 ml DMSO werden auf 100°C erwärmt und über 1 Stunde tropfenweise mit 9,2 g (0,15 Mol) Nitromethan in 10 ml DMSO versetzt. Man läßt 2 Stunden nachreagieren und arbeitet analog Beispiel 2 auf.
Man erhält 17,6 g Rückstand mit 26% 1-Nitromethyl-cyclohexanessigsäureäthylester, 30,2% Ausgangsverbindung und 35,9% Nebenprodukt (siehe Beispiel 3).

### Beispiel 6

16,8 (0,1 Mol) Cyclohexylidenessigsäureäthylester und 2 g (0,01 Mol) Tetraäthylammoniumfluorid·2 H₂O wurden in 40 ml DMSO auf 100°C erwärmt und anschließend über 1 h kontinuierlich mit 9,2 g (0,15 Mol) Nitromethan versetzt. Man läßt 2 h bei 100°C nachreagieren und arbeitet analog Beispiel 2 auf. Man erhält 15,4 g öligen Rückstand, enthaltend ca. 10% Stufe 2 und ca. 90% 1-Cyclohexen-essigsäureäthylester (Vgl. Beispiel 3).

### Beispiel 7

16,8 g (0,1 Mol) Cyclohexylidenessigsäureäthylester und 4 g (0,02 Mol) Tetraäthylammouniumfluorid·2 H₂O werden in 50 ml Nitromethan 3 Stunden unter Rückfluß gerührt. Anschließend wird die Reaktionsmischung mit 100 ml verdünnter Salzsäure versetzt und mit Dichlormethan mehrmals extrahiert. Die organischen Phasen werden neutral gewaschen und getrocknet. Nach Entfernen des Lösungsmittels im Vakuum erhält man als öligen Rückstand 20,9 g (91,3% der Theorie) mit einem Gehalt von 84% Stufe 2 (GC)

### Beispiel 8

### Herstellung von 2-Aza Spiro 4,5 decan-3-on (Stufe 3)

190 g (0,82 Mol) 1-Nitromethyl-cyclohexanessigsäureäthylester in 3168 ml Äthanol werden mit 62,9 g Palladium-Kohle (10%ig) bei 125°C in 4 Stunden hydriert.
Nach beendeter Wasserstoffaufnahme wird vom Katalysator abfiltriert und die erhaltene farblose Lösung im Vakuum zur Trockne abdestilliert Man erhält 116,2 g 2-Aza-Spiro [4,5] decan-3-on als farbloses Kristallisat (91,6% der Theorie)
GC-Gehalt 97,1%, Schmp. 88-90°C

### Beispiel 9

### Herstellung von 1-Aminomethyl-1-cyclohexanessigsäure-Hydrochlorid (Stufe 4)

89 g (0,581 Mol) 2-Aza-spiro [4,5] decan-3-on werden mit halbkonzentrierter Salzsäure 4 Stunden unter Rückfluß gerührt. Das auf Raumtemperatur abgekühlte Reaktionsgemisch wird mit Wasser verdünnt und mit Methylenchlorid zur Entfernung der Vorstufe ausgeschüttelt. Die wässrige Phase wird im Vakuum bis zur Trockne eingeengt und der erhaltene Rückstand mit Aceton versetzt.
Beim Stehen kristallisieren 79,85 g 1-Aminomethyl-1-cyclohexanessigsäure·HCl (64,7% der Theorie), Gehalt 99%, Schmp.: 123-127°C
Aus der Mutterlauge lassen sich durch Einengen nochmals 6,2 g Produkt isolieren (5% der Theorie). Aus der Methylenchloridphase wird durch Entfernen des Lösungsmittels nicht umgesetztes 2-Aza-spiro [4,5] decan-3-on (20-25 g) zurückgewonnen und erneut mit halbkonzentrierter Salzsäure umgesetzt.

### Beispiel 10

### Herstellung von 1-Aminomethyl-1-cyclohexanessigsäure (Stufe 5)

2 kg (9,42 Mol) 1-Aminomethyl-1-cyclohexanessigsäure-Hydrochlorid werden in Wasser gelöst und über eine mit Ionenaustauscher gefüllte Säule eluiert. Die erhaltene wässrige Lösung der freien Aminosäure wird schonend im Vacuum zur Trockne eingeengt und der Rückstand aus Methanol kristallisiert.
Man erhält 1,339 kg 1-Aminomethyl-1-cyclohexanessigsäure (83% der Theorie). Schmp: 165-167°C. Reinheit > 99,5 %

### Herstellung von Cyclohexylidenessigsäureäthylester (Stufe 1)

### Beispiel 11

3.66 kg (37.29 mol) Cyclohexanon werden mit 8.87 kg (39.56 mol) Phosphonoessigsäuretriäthylester gemischt. Unter Kühlung wird das Gemisch portionsweise so mit 2.51 kg (38.02 mol) KOH-Pulver (Gehalt 85%) versetzt, daß eine Temperatur von 30 bis 40° C ein gehalten wird. Nach einer Stunde wird das Reaktionsgemisch mit 18 l Wasser versetzt und anschließend mehrfach mit n-Hexan extrahiert. Die vereinigten organischen Phasen werden mit Wasser gewaschen, über Natriumsulfat getrocknet und dann im Vakuum das Lösungsmittel abdestilliert.
Man erhält 5.78 kg Cyclohexylidenessigsäureäthylester als Öl.
(92.1 % der Theorie).
Gehalt nach HPLC und GC (96.5 - 99.5 %).

### Beispiel 12

Ein Gemisch aus 5.00 kg (50.94 mol) Cyclohexanon und 12.00 kg (53.53 mol) Phosphonoessigsäuretriäthylester wird mit 3.36 kg (52.70 mol) KOH-Pulver (Gehalt 88 %) portionsweise versetzt. Es wird eine Temperatur von 30 - 40 °C eingehalten. Nach beendeter Zugabe läßt man noch 1 Stunde rühren. Der Ansatz wird in zwei Hälften zu je 10.15 Kg geteilt.
a) 10.15 kg werden wie im Beispiel 1 beschrieben aufgearbeitet. Man erhält 3.92 kg Cyclohexylidenessigsäureäthylester als Öl.
   (91.5 % der Theorie)
   Gehalt nach HPLC 99.8 %.
b) 10.15 kg Reaktionsgemisch werden mit 9.8 l DMSO versetzt. 16.06 kg dieser Mischung enthalten ca. 18 mol Stufe 1 und werden zur Umsetzung im Reaktionsschritt 2 eingesetzt.
   (Beispiel 3)

### Herstellung von 1-Nitromethyl-cyclohexanessigsäureäthylester (Stufe 2)

### Beispiel 13

1.294 kg (9.36 mol) Kaliumcarbonat werden in 8 l DMSO suspendiert und auf 110 - 115 °C erwärmt. 16.06 kg DMSO-Lösung der Stufe 1 (wie unter Beispiel 2b beschrieben; entsprechend 18 mol Stufe 1) werden mit 1.72 kg (28.18 mol) Nitromethan versetzt und im Verlauf von 3.5 Stunden bei 110 - 115 °C in die Kaliumcarbonat-DMSO Suspension getropft. Man läßt 4 Stunden bei 110 °C nachrühren und über Nacht auf Raumtemperatur abkühlen.
Der Reaktionslösung wurden 2.50 kg für analytische Zwecke entnommen.
Die restliche Mischung wird mit 40 l Wasser versetzt und anschließend mehrfach mit n-Hexan extrahiert. Die vereinigten organischen Phasen werden mit Wasser gewaschen, über Natriumsulfat getrocknet und dann im Vakuum das Lösungsmittel abdestilliert. Man erhält 3.38 kg 1-Nitromethyl-cyclohexanessigsäureäthylester als Öl (83 % der Theorie).
Gehalt nach HPLC: 86.5 % Stufe 2 und 4.5 % Stufe 1

### Beispiel 14

98.15 g (1.00 mol) Cyclohexanon werden mit 235.4 g (1.05 mol) Phosphonsäureäthylester gemischt und bei 25 - 30 °C mit 66.1 g (1.04 mol) KOH-Pulver (Gehalt 88 %) versetzt. Man läßt noch 30 min rühren, versetzt das Gemisch nacheinander mit 150 ml DMSO und 86.0 g (1.41 mol) Nitromethan. Diese Lösung wird bei 110 - 115 °C im Verlauf von 35 min in eine Suspension von 64.7 g (0.47 mol) Kaliumcarbonat in 470 ml DMSO getropft. Nach beendeter Zugabe wird 2- 3 Stunden nachgerührt, die Reaktionsmischung mit Wasser versetzt und anschließend mehrfach mit n-Hexan extrahiert. Die vereinigten organischen Phasen werden mit Wasser gewaschen, über Natriumsulfat getrocknet und dann im Vakuum das Losungsmittel abdestilliert.
Man erhält 191.5 g 1-Nitromethyl-cyclohexanessigsäureäthylester als Öl (83 % der Theorie).
Zusammensetzung nach HPLC: 88.7 % Stufe 2 und 6.3% Stufe 1

### Herstellung von 2-Aza-spiro [4,5] decan-3-on (Stufe 3)

### Beispiel 15

30 g (0.13 mol) 1-Nitromethyl-cyclohexanessigsäureäthylester (Gehalt nach GC 83 %) in 300 ml Ethanol werden mit 3 g Palladium-Kohle (10 % ig) bei 60 - 80 °C und 0.6 bar in 3 Stunden hydriert. Nach beendeter Wasserstoffaufnahme wird vom Katalysator abfiltriert und die fast farblose Lösung im Vakuum zur Trockne abdestilliert. Man erhält als hellbraunes Kristallisat 19.0 g 2-Aza-spiro[4,5]decan-3-on ( 95 % der Theorie ).
Gehalt nach GC: 83 %

## Patentansprüche

1. Verfahren zur Herstellung von 1-Aminomethyl-1-cyclohexanessigsäure nach dem Prinzip der Michael-Addition gekennzeichnet durch folgende Reaktionsschritte:
a.) Umsetzung von Cyclohexanon mit einer Phosphonsäureester-Verbindung zu Cyclohexylidenessigsäureester
b.) Umsetzung des Cyclohexylidenessigsäureesters mit Nitromethan zu 1-Nitromethyl-cyclohexanessigsäureester
c.) Reduktion des 1-Nitromethyl-cyclohexanessigsäureesters zu 1-Aminomethyl-cyclohexanessigsäureester und 2-Aza-Spiro [4,5] decan-3-on
d.) Umwandlung der im Schritt c.) entstandenen Produkte in 1-Aminomethyl-1-cyclohexanessigsäure-Salze mittels verdünnter Säuren.
e.) Freisetzung der 1-Aminomethyl-1-cyclohexanessigsäure aus dem Salz mittels Ionenaustauschern,
wobei in Schritt a.) zunächst das Cyclohexanon bei Raumtemperatur mit KOH umgesetzt wird, bevor der Phosphonsäureester zugegeben wird,
in Schritt b.) DMSO als Lösungsmittel und ein Alkalimetallcarbonat als Katalysator verwendet wird
in Schritt c.) bei erhöhter Temperatur, nämlich oberhalb 100°C gearbeitet wird.

## Claims

1. Process for the preparation of 1-aminomethyl-1-cyclohexaneacetic acid according to the principle of a Michael addition, characterised by the following steps:
a.) reaction of cyclohexanone with a phosphonic acid ester compound to give a cyclohexylideneacetic acid ester;
b.) reaction of the cyclohexylideneacetic acid ester with nitromethane to give 1-nitromethyl-1-cyclohexaneacetic acid ester;
c.) reduction of the 1-nitromethyl-cyclohexaneacetic acid ester to 1-aminomethyl-cyclohexaneacetic acid ester and 2-aza-spiro[4,5]-decan-3-one;
d.) conversion of the products obtained in step c.) into 1-aminomethyl-1-cyclohexaneacetic acid salts by means of dilute acids;
e.) liberation of 1-aminomethyl-1-cylcohexaneacetic acid from the salt by means of ion exchangers,
wherein in step a.) the cyclohexanone is first reacted at ambient temperature with potassium hydroxide before the phosphonic acid ester is added,
in step b.) dimethyl sulphoxide is used as solvent and an alkali metal carbonate is used as catalyst,
in step c.) working is carried out at an elevated temperature above 100°C.

## Revendications

1. Procédé de préparation d'acide 1-aminométhyl-1-cyclohexane-acétique suivant le principe de l'addition de Michael, caractérisé par les étapes de réaction suivantes:
a) réaction de la cyclohexanone avec un composé d'ester d'acide phosphonique pour former un ester de l'acide cyclohexylidène-acétique
b) réaction de l'ester de l'acide cyclohexylidène acétique avec le nitrométhane pour former l'ester de l'acide 1-nitrométhyl-cyclohexane acétique
c) réduction de l'ester de l'acide 1-nitrométhyl-cyclohexane-acétique en ester de l'acide 1-aminométhyl-cyclohexane-acétique et en 2-aza-spiro[4,5]décane-3-one
d) transformation des produits obtenus à l'étape c) en sels de l'acide 1-aminométhyl-1-cyclohexane-acétique au moyen d'acides dilués
e) séparation de l'acide 1-aminométhyl-1-cyclohexane-acétique de son sel par échange d'ions
tandis qu'à l'étape a), la cyclohexanone réagit tout d'abord à température ambiante avec KOH avant l'addition de l'ester de l'acide phosphonique,
qu'à l'étape b) on utilise comme solvant du DMSO et comme catalyseur un carbonate de métal alcalin,
qu'à l'étape c) on opère à température élevée, c'est-à-dire au-dessus de 100°C.
